# EUROPEAN PATENT APPLICATION

(11) **EP 4 418 303 A2**
(43) Date of publication of application: **21.08.2024**
(21) Application number: 24153246.4
(22) Date of filing: 22.01.2024
(51) Int. Cl.: H01J 61/12, F21V 7/00, F21V 23/04, H01J 61/16, H01J 65/04

(54) **ULTRAVIOLET LIGHT EMISSION DEVICE**

(30) Priority: 27.01.2023 JP 2023010698
(71) Applicant: Ushio Denki Kabushiki Kaisha, Tokyo 100-8150 (JP)
(72) Inventor: YOSHIHARA, Keita, Tokyo, 100-8150 (JP); SATO, Shingo, Tokyo, 100-8150 (JP); ISHIHARA, Hajime, Tokyo, 100-8150 (JP)
(74) Representative: Maiwald GmbH

(57) **Abstract**

An ultraviolet light emission device (1) includes: an excimer lamp (2) having an elongated shape and having a light emission surface (2a) that emits ultraviolet light (L) toward an object (W) for irradiation; a light intensity sensor (3) that is disposed around the excimer lamp (2) and detects ultraviolet light (L); and a first reflection member (61) disposed around the excimer lamp (2) and facing a part of the light emission surface (2a) in a longitudinal direction, wherein ultraviolet light (L) emitted from the light emission surface (2a) is reflected by the first reflection member (61) and enters the light intensity sensor (3).

## Description

### BACKGROUND OF THE INVENTION

### Field of the Invention

The present invention relates to an ultraviolet light emission device.

### Description of the Related Art

Conventionally, in a manufacturing process of semiconductor substrates or liquid crystal substrates, an ultraviolet light emission device using, as a light source, an excimer lamp that emits ultraviolet light for the purpose of cleaning substrates is known.

A light-emitting tube of the excimer lamp has a characteristic that a transmission wavelength ranges of ultraviolet light changes depending on temperatures, that is, a wavelength characteristic of ultraviolet light emitted from the excimer lamp varies depending on the temperature of the light-emitting tube. This characteristic is very tricky in stabilizing a process and leads to a limited use of the ultraviolet light emission device. In order to address this problem, feedback control is known in which ultraviolet light emitted from an excimer lamp is constantly monitored and input power is adjusted in accordance with the intensity of ultraviolet light (for example, Patent Document 1).

### Prior Art Document

### Patent Document

Patent Document 1: JP-A-2004-041843
Patent Document 2: JP-B2-5287928
Patent Document 3: JP-A-2021-157902

### SUMMARY OF THE INVENTION

In Patent Document 2, a lighting port is provided in a part of a reflective film formed on an inner surface of a discharge container, and ultraviolet light emitted from the lighting port is detected by an optical monitor. In Patent Document 2, it is necessary to change the structure of a lamp only in accordance with the position of the optical monitor as described above. However, according to this configuration, a portion that detects ultraviolet light and a portion that is used for actual workpiece processing are different in structure, and thus, the performance of feedback control is deteriorated.

In Patent Document 3, a workpiece is irradiated with light using a reflective film, and light reflected from the reflective film is detected by a light intensity sensor disposed on a side opposite to the workpiece. Commonly, the outer wall surface of a light-emitting tube on the side opposite to the workpiece is cooled, and thus, the temperature of the outer wall surface on the side opposite to the workpiece is lower than the temperature of the outer wall surface on the workpiece side. Therefore, light emitted to the side opposite to the workpiece and light emitted to the workpiece side are different in wavelength characteristics, so that the performance of feedback control is deteriorated.

In view of the above problems, an object of the present invention is to provide an ultraviolet light emission device capable of improving performance of feedback control.

An ultraviolet light emission device according to the present invention includes:
an excimer lamp having an elongated shape and having a light emission surface that emits ultraviolet light toward an object for irradiation;
a light intensity sensor that is disposed around the excimer lamp and detects ultraviolet light; and
a first reflection member disposed around the excimer lamp and facing a part of the light emission surface in a longitudinal direction, wherein
ultraviolet light emitted from the light emission surface is reflected by the first reflection member and enters the light intensity sensor.

According to this configuration, ultraviolet light emitted from the light emission surface toward the object for irradiation can be directly detected by the light intensity sensor, whereby the light intensity detected by the light intensity sensor can be made substantially equal to the intensity of light emitted from the light emission surface and applied to the object for irradiation. As a result, the performance of feedback control of the ultraviolet light emission device can be enhanced.

In the ultraviolet light emission device, the excimer lamp may include a light-emitting tube having transparency to ultraviolet light, and a reflective film that is formed on an inner wall surface of the light-emitting tube and that faces the light emission surface across a tube axis of the light-emitting tube.

Ultraviolet light emitted from the light emission surface includes ultraviolet light emitted from the inside of the light-emitting tube and traveling toward the light emission surface and ultraviolet light reflected toward the light emission surface by the reflective film. Meanwhile, the ultraviolet light emitted from the light emission surface toward the object for irradiation can be directly detected by the light intensity sensor. Therefore, no problem occurs even if there is a change in light output over time due to, for example, deterioration of the reflective film.

The ultraviolet light emission device may further include a second reflection member disposed around the excimer lamp and located lateral to the light emission surface, wherein ultraviolet light reflected by the first reflection member is reflected by the second reflection member and enters the light intensity sensor.

The ultraviolet light emission device may further include a third reflection member disposed between the excimer lamp and the light intensity sensor, wherein ultraviolet light reflected by the second reflection member is reflected by the third reflection member and enters the light intensity sensor.

According to these configurations, the light intensity sensor is easily disposed around the excimer lamp and on the side opposite to the object for irradiation across the excimer lamp, whereby the light emission surface can be brought close to the object for irradiation.

In the ultraviolet light emission device, the light intensity sensor may face the first reflection member across the excimer lamp.

According to this configuration, the light intensity sensor can be disposed around the excimer lamp and on the side opposite to the object for irradiation across the excimer lamp.

In the ultraviolet light emission device, it is preferable that a space between the periphery of the excimer lamp and the light intensity sensor has an inert gas atmosphere.

This configuration can suppress a decrease in intensity of ultraviolet light emitted from the light emission surface before the ultraviolet light enters the light intensity sensor, and thus, can enhance the performance of feedback control.

In the ultraviolet light emission device,
the light emission surface may be a lower surface of the light-emitting tube, and
the ultraviolet light emission device may include a plurality of the excimer lamps that is arranged in a lateral direction such that the tube axes of the light-emitting tubes are parallel to each other, and a plurality of the light intensity sensors each of which is disposed above the corresponding one of the excimer lamps.

According to this configuration, a plurality of excimer lamps can be densely arranged, whereby it is easy to uniformly irradiate the object for irradiation with ultraviolet light.

In the ultraviolet light emission device, the second reflection member may be preferably disposed between the excimer lamps adjacent to each other.

This configuration can prevent the entry of ultraviolet light into the light intensity sensor disposed around a certain excimer lamp from another excimer lamp adjacent to the certain excimer lamp, and thus, can enhance the performance of feedback control for the certain excimer lamp.

### BRIEF DESCRIPTION OF THE DRAWINGS

Fig. 1 is a front view schematically illustrating a configuration of an ultraviolet light emission device according to an embodiment of the present invention;
Fig. 2 is a schematic cross-sectional view of an excimer lamp cut along a plane including a tube axis;
Fig. 3 is a schematic cross-sectional view of the excimer lamp cut along a plane perpendicular to the tube axis;
Fig. 4 is a perspective view of the excimer lamp and a cooling block;
Fig. 5 is a cross-sectional view taken along line V-V in Fig. 4;
Fig. 6 is a cross-sectional view taken along line VI-VI in Fig. 4;
Fig. 7 is a cross-sectional view taken along line VII-VII in Fig. 1;
Fig. 8 is a partially enlarged view of Fig. 7; and
Fig. 9 is a schematic cross-sectional view of an excimer lamp and a cooling block cut along a plane perpendicular to a tube axis according to another embodiment.

### DETAILED DESCRIPTION OF THE PREFERRED EMBODIMENTS

Embodiments of an ultraviolet light emission device according to the present invention will be described with reference to the drawings. Note that the following drawings are schematically illustrated, the dimensional ratios in the drawings do not necessarily coincide with the actual dimensional ratios, and the dimensional ratios do not necessarily coincide between the drawings.

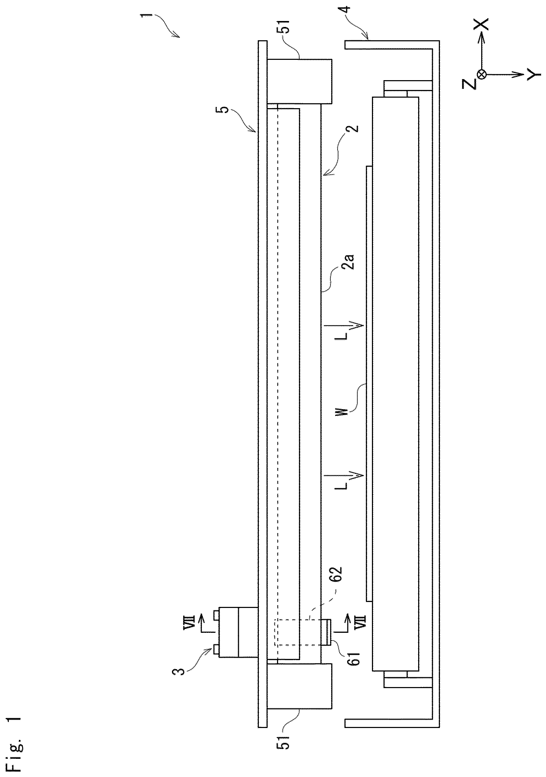

Fig. 1 is a front view schematically illustrating a configuration of an ultraviolet light emission device according to one embodiment. As illustrated in Fig. 1, an ultraviolet light emission device 1 includes an excimer lamp 2 and a light intensity sensor 3. The ultraviolet light emission device 1 irradiates an object W for irradiation placed on a light emission surface 2a side of the excimer lamp 2 with ultraviolet light.

The ultraviolet light emission device 1 may include a conveyance mechanism 4 that conveys the object W. The conveyance mechanism 4 conveys the placed object W and irradiates the object W with ultraviolet light emitted from the light emission surface 2a of the excimer lamp 2. The object W irradiated with ultraviolet light is carried out of the conveyance mechanism 4.

The conveyance mechanism 4 in the present embodiment is constituted by a plurality of rollers spaced from each other, but may have any configuration such as a belt-shaped conveyor, as long as the conveyance mechanism 4 can convey the object W in a predetermined direction while maintaining the distance between the excimer lamp 2 and the object W.

In the following description, a direction (tube axis direction) in which the excimer lamp 2 extends is defined as an X direction, a direction from the light emission surface 2a of the excimer lamp 2 to the object W and perpendicular to the X direction is defined as a Y direction, and a direction orthogonal to the X direction and the Y direction is defined as a Z direction, as illustrated in Fig. 1. Furthermore, positive and negative orientations distinguished from each other for directional expression will be described as a "+X direction" and a "-X direction" by adding positive and negative signs, while a direction expressed without distinction between positive and negative orientations will be described simply as the "X direction". The ultraviolet light emission device 1 according to the present embodiment is disposed with the Y direction as the vertical direction.

First, the configuration of the excimer lamp 2 will be described. The excimer lamp 2 is disposed such that the light emission surface 2a faces the conveyance mechanism 4 so as to irradiate the object W with ultraviolet light.

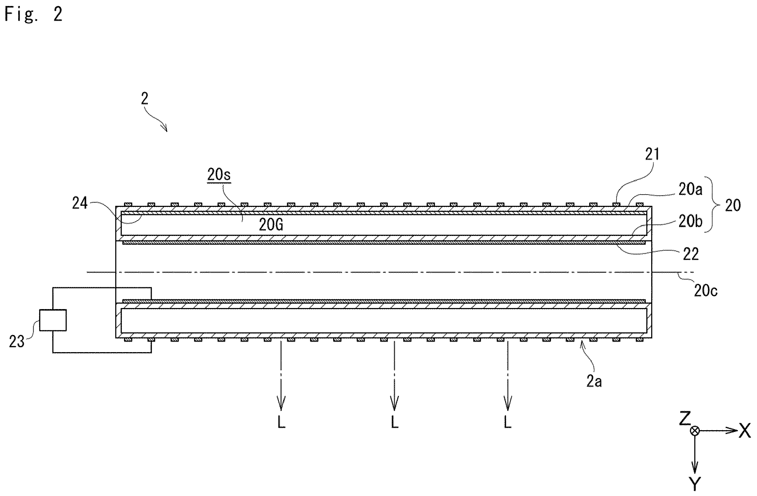

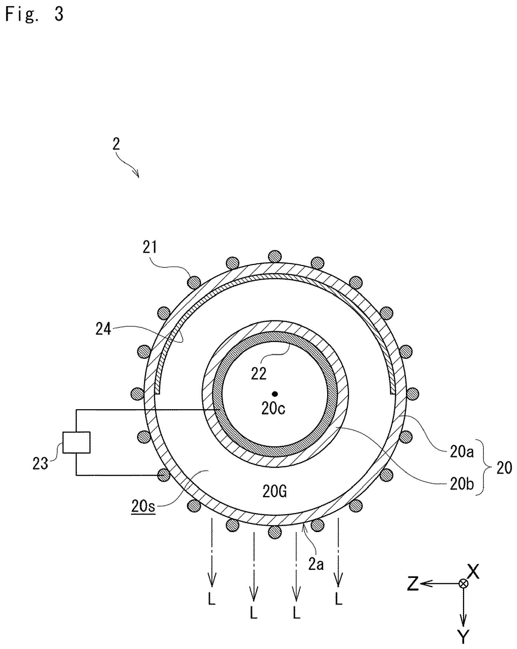

Fig. 2 is a schematic cross-sectional view of the excimer lamp 2 cut along a plane including a tube axis 20c. Fig. 3 is a schematic cross-sectional view of the excimer lamp 2 cut along a plane perpendicular to the tube axis 20c. The excimer lamp 2 includes a light-emitting tube 20 extending along the X direction. More specifically, the light-emitting tube 20 has an outer tube 20a that is cylindrical and positioned outside, and an inner tube 20b that is cylindrical and disposed inside the outer tube 20a, coaxial with the outer tube 20a, and having an inner diameter smaller than that of the outer tube 20a. The outer tube 20a and the inner tube 20b are both made of a dielectric material such as synthetic quartz glass.

The outer tube 20a and the inner tube 20b are sealed at the ends in the X direction, so that a discharge space 20s that is annular when viewed in the X direction is formed between both tubes. A discharge gas 20G that generates excimer molecules by an electric discharge is sealed in the discharge space 20s. A more specific example of the discharge gas 20G is a gas mixture containing xenon (Xe) and neon (Ne) in a predetermined ratio (for example, 3:7), which may or may not further include a small amount of oxygen or hydrogen.

The excimer lamp 2 in the present embodiment includes a first electrode 21 set on an outer wall surface of the outer tube 20a, and a second electrode 22 set on an inner wall surface of the inner tube 20b. The first electrode 21 has a mesh pattern or a line pattern. The second electrode 22 has a film shape. The second electrode 22 may have a mesh pattern or a line pattern as in the first electrode 21.

A lighting power supply 23 applies a high-frequency AC voltage of, for example, about 50 kHz to 5 MHz between the first electrode 21 and the second electrode 22. In the present embodiment, the lighting power supply 23 is configured to apply a periodic pulsed voltage to the first electrode 21 with the second electrode 22 as a reference potential. The magnitude (absolute value) of the voltage is high on the order of kV at 0-peak, and is preferably 1 kV or more and 9 kV or less.

When the lighting power supply 23 applies a high-frequency AC voltage between the first electrode 21 and the second electrode 22, the applied voltage is applied to the discharge gas 20G through the light-emitting tube 20, so that a discharge plasma is generated in the discharge space 20s filled with the discharge gas 20G. As a result, atoms of the discharge gas 20G are excited into the excimer state, and excimer emission occurs when the atoms are transferred to the ground state. In a case where the above-mentioned gas containing xenon (Xe) is used as the discharge gas 20G, the excimer emits ultraviolet light L that has a wavelength around 172 nm. The wavelength of the ultraviolet light L can be changed by using a different substance for the discharge gas 20G. For example, ArBr (165 nm), ArCI (175 nm), F₂ (153 nm), KrCI (222 nm), XeCI (308 nm), XeBr (283 nm), KrBr (207 nm), or the like can be used as the discharge gas 20G.

As described above, the first electrode 21 has a mesh pattern or a line pattern so as not to inhibit emission of the ultraviolet light L generated inside the light-emitting tube 20 (more specifically, inside the discharge space 20s) to the outside of the light-emitting tube 20. Thus, the ultraviolet light L is emitted to the outside of the light-emitting tube 20 through the clearance of the first electrode 21.

The excimer lamp 2 includes the light emission surface 2a. The light emission surface 2a is a partial region of a wall surface of the light-emitting tube 20 that emits the ultraviolet light L emitted from the discharge space 20s of the light-emitting tube 20 to the object W. In the present embodiment, the light emission surface 2a is a surface facing the +Y direction (downward direction) of the wall surface of the light-emitting tube 20.

In addition, the excimer lamp 2 includes a reflective film 24 facing the light emission surface 2a across the tube axis 20c of the light-emitting tube 20. The reflective film 24 is formed on, for example, the inner wall surface of the outer tube 20a. Examples of a constituent material usable for the reflective film 24 include a material formed by applying a suspension or the like containing particulate silica (SiOz), alumina (Al₂O₃), or the like and firing the applied suspension or the like.

The ultraviolet light L emitted from the discharge space 20s and traveling toward the light emission surface 2a directly passes through the clearance of the first electrode 21 and is emitted toward the outside of the light-emitting tube 20. On the other hand, the ultraviolet light L emitted from the discharge space 20s and traveling to the side opposite to the light emission surface 2a, that is, in the -Y direction is reflected by the reflective film 24 to travel to the light emission surface 2a side, that is, in the +Y direction, passes through the clearance of the first electrode 21, and is emitted toward the outside of the light-emitting tube 20.

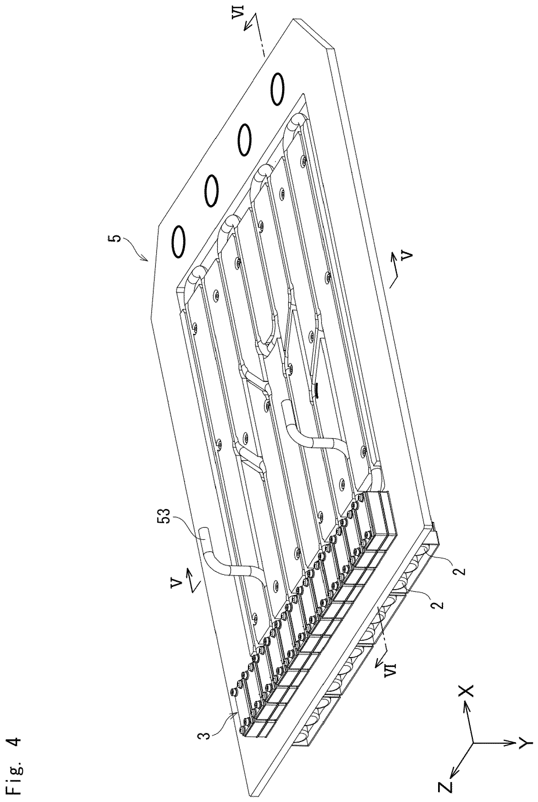

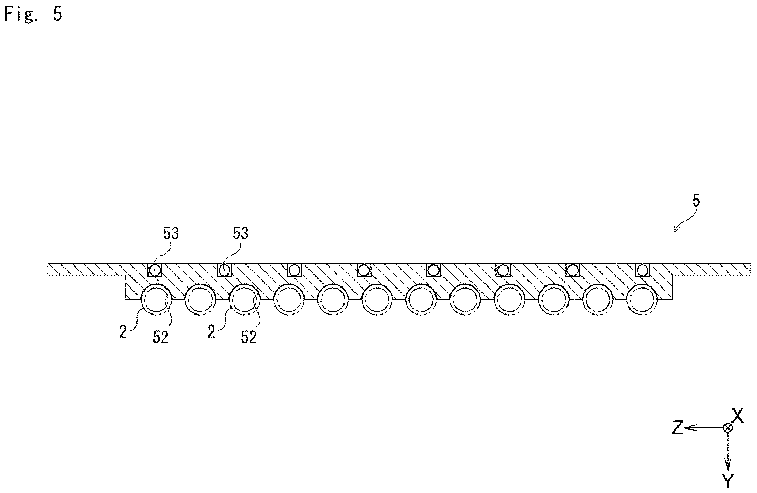

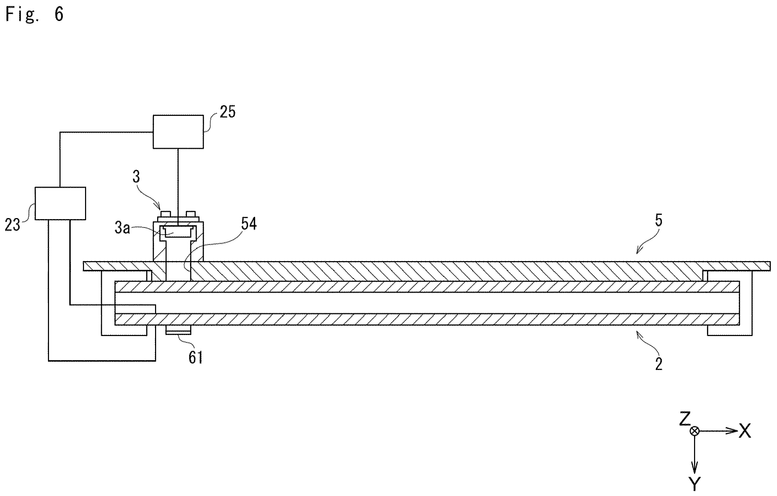

The excimer lamp 2 is held on a cooling block 5 with a pair of base portions 51 as illustrated in Fig. 1. Fig. 4 is a perspective view of the excimer lamp 2 and the cooling block 5. Fig. 5 is a cross-sectional view taken along line V-V in Fig. 4, and Fig. 6 is a cross-sectional view taken along line VI-VI in Fig. 4.

The cooling block 5 has a substantially rectangular plate shape and has a plurality of grooves 52 extending in the X direction in parallel with each other in the lower surface, each of the grooves 52 having a semicircular cross section. Each groove 52 has an inner diameter substantially equal to the outer diameter of the excimer lamp 2, and the excimer lamp 2 is disposed and housed in the corresponding groove 52. That is, a plurality of excimer lamps 2 is arranged side by side in the Z direction such that the tube axes 20c of the light-emitting tubes 20 are parallel to each other. In the present embodiment, twelve excimer lamps 2 are arranged side by side.

Cooling water pipes 53 are embedded in the upper surface of the cooling block 5, and the excimer lamps 2 can be cooled through the grooves 52 by circulating cooling water in the cooling water pipes 53. Thus, the light-emitting tube 20 of the excimer lamp 2 has a large temperature difference between the surface (upper surface) on the cooling block 5 side and the surface (lower surface) on the light emission surface 2a side. The transmittance characteristics of synthetic quartz glass vary depending on temperatures.

In addition, the cooling block 5 is formed with through holes 54 extending along the Y direction so as to face the outer wall surfaces of the excimer lamps 2 (the outer wall surfaces of the outer tubes 20a) housed in the grooves 52 as illustrated in Fig. 6. The light intensity sensors 3 are disposed on the upper surface of the cooling block 5 so as to close the upper openings of the through holes 54. The light intensity sensor 3 incorporates a light receiving unit 3a, and the light receiving unit 3a faces the outer wall surface of the excimer lamp 2 via the through hole 54. The light intensity sensor 3 may employ, for example, a silicon photodiode. The light intensity sensor 3 detects the ultraviolet light L emitted from the light emission surface 2a (which will be described in detail below.

The ultraviolet light emission device 1 also includes a control device 25 connected to the light intensity sensors 3. The control device 25 performs feedback control on an input current supplied to the excimer lamp 2 from the lighting power supply 23 on the basis of the light intensity detected by the light intensity sensor 3 so that the light intensity detected by the light intensity sensor 3 is constant.

From the viewpoint of enhancing the performance of the feedback control, it is preferable to bring the light intensity detected by the light intensity sensor 3 as close as possible to the intensity of light emitted from the light emission surface 2a to the object W. That is, it is preferable that the ultraviolet light L emitted to the object W is directly detected by the light intensity sensor 3. However, due to the presence of the conveyance mechanism 4 on the light emission surface 2a side of the excimer lamp 2, it is difficult to dispose the light intensity sensor 3 on the light emission surface 2a side, and a structure in which the light intensity sensor 3 is disposed on a side other than the light emission surface 2a side of the excimer lamp 2 is desired.

The ultraviolet light emission device 1 includes a first reflection member 61 disposed around the excimer lamp 2 and facing a part of the light emission surface 2a in the longitudinal direction (X direction). The first reflection member 61 in the present embodiment faces a region near the end of the light emission surface 2a on the -X direction side in the Y direction as illustrated in Fig. 1.

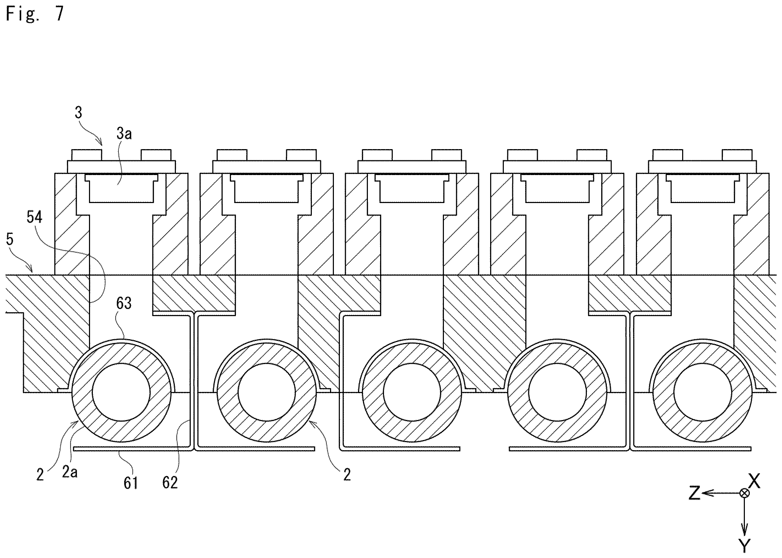

Fig. 7 is a cross-sectional view taken along line VII-VII in Fig. 1. The first reflection member 61 is disposed away from the excimer lamp 2. The first reflection member 61 faces the through hole 54 of the cooling block 5 in the Y direction. The first reflection member 61 is disposed to be shifted in the X direction so as not to overlap with the object W when viewed in the Y direction as illustrated in Fig. 1. The light intensity sensor 3 faces the first reflection member 61 across the excimer lamp 2.

In the present embodiment, the first reflection member 61 is a rectangular plate-shaped member disposed below the excimer lamp 2. The first reflection member 61 extends in the X direction and the Z direction. In the first reflection member 61, a surface facing the light emission surface 2a is at least a reflection surface.

The ultraviolet light emission device 1 also includes a second reflection member 62 disposed around the excimer lamp 2 and located lateral to the light emission surface 2a. The second reflection member 62 is disposed away from the excimer lamp 2. The second reflection member 62 in the present embodiment faces a region near the end of the light emission surface 2a on the -X direction side in the Z direction as illustrated in Fig. 1.

When a plurality of excimer lamps 2 is arranged side by side as in the present embodiment, the second reflection member 62 is disposed between the excimer lamps 2 adjacent to each other. This configuration can prevent the entry of the ultraviolet light L into the light intensity sensor 3 disposed around a certain excimer lamp 2 from another excimer lamp 2 adjacent to the certain excimer lamp 2. As a result, the performance of the feedback control for a certain excimer lamp 2 can be improved.

In the present embodiment, the second reflection member 62 is a rectangular plate-shaped member disposed lateral to the excimer lamp 2. The second reflection member 62 extends in the X direction and the Y direction. In the second reflection member 62, a surface facing the light emission surface 2a is at least a reflection surface.

The second reflection member 62 may be formed integrally with the first reflection member 61 as in the present embodiment. In the present embodiment, the first reflection member 61 and the second reflection member 62 have an L shape as a whole.

The ultraviolet light emission device 1 also includes a third reflection member 63 disposed between the excimer lamp 2 and the light intensity sensor 3. The third reflection member 63 in the present embodiment faces the light receiving unit 3a of the light intensity sensor 3 in the Y direction.

In the present embodiment, the third reflection member 63 is a semi-cylindrical member disposed on the outer wall surface of the light-emitting tube 20 of the excimer lamp 2. In the third reflection member 63, a surface facing the light receiving unit 3a is at least a reflection surface.

The material of the first to third reflection members 61 to 63 is, for example, aluminum or silver, preferably bright aluminum.

Next, a process of detecting the ultraviolet light L emitted from the light emission surface 2a of the excimer lamp 2 by the light intensity sensor 3 will be described.

The ultraviolet light L emitted from the light emission surface 2a is reflected by the first reflection member 61 and is directed to the second reflection member 62. The ultraviolet light L directed to the second reflection member 62 is then reflected by the second reflection member 62 and directed to the third reflection member 63. The ultraviolet light L directed to the third reflection member 63 is then reflected by the third reflection member 63 and directed to the light receiving unit 3a of the light intensity sensor 3. As described above, the ultraviolet light L emitted from the light emission surface 2a is sequentially reflected by the first reflection member 61, the second reflection member 62, and the third reflection member 63, and enters the light intensity sensor 3. Thus, the ultraviolet light L emitted from the light emission surface 2a can be directly detected by the light intensity sensor 3, whereby the light intensity detected by the light intensity sensor 3 can be the same as or equal to the intensity of light emitted from the light emission surface 2a and applied to the object W. As a result, the performance of the feedback control of the ultraviolet light emission device 1 can be enhanced.

Preferably, a space between the periphery of the excimer lamp 2 and the light intensity sensor 3 has an inert gas atmosphere. The inert gas is, for example, nitrogen gas. This configuration can suppress a decrease in intensity of ultraviolet light L emitted from the light emission surface 2a before the ultraviolet light L enters the light intensity sensor 3 after being reflected by the first reflection member 61, the second reflection member 62, and the third reflection member 63, and thus, can enhance the performance of the feedback control. It is also preferable that the space between the excimer lamp 2 and the object W similarly has an inert gas atmosphere.

While the embodiment of the present invention has been described above with reference to the drawings, it should be construed that specific configurations are not limited to the above embodiment. The scope of the present invention is indicated not only by the above description of the embodiments but also by the claims, and all changes which come within the meaning and range of equivalency of the claims are therefore intended to be embraced therein.

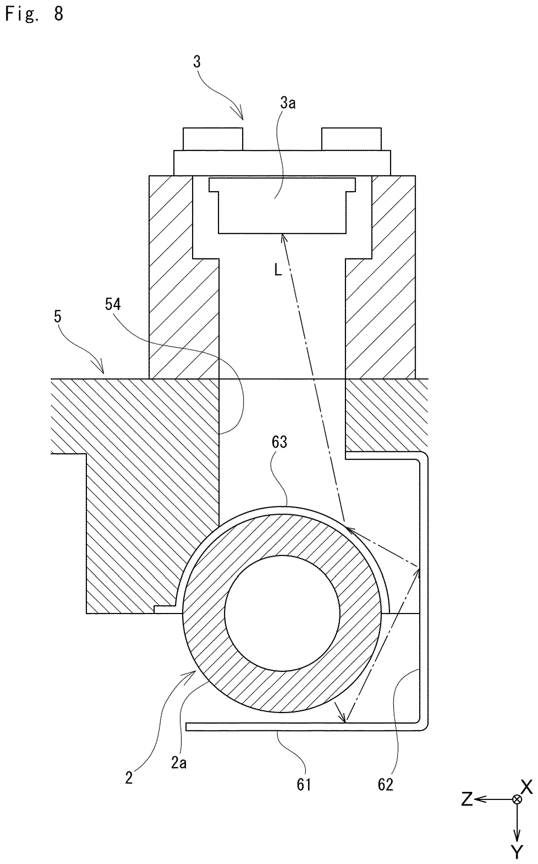

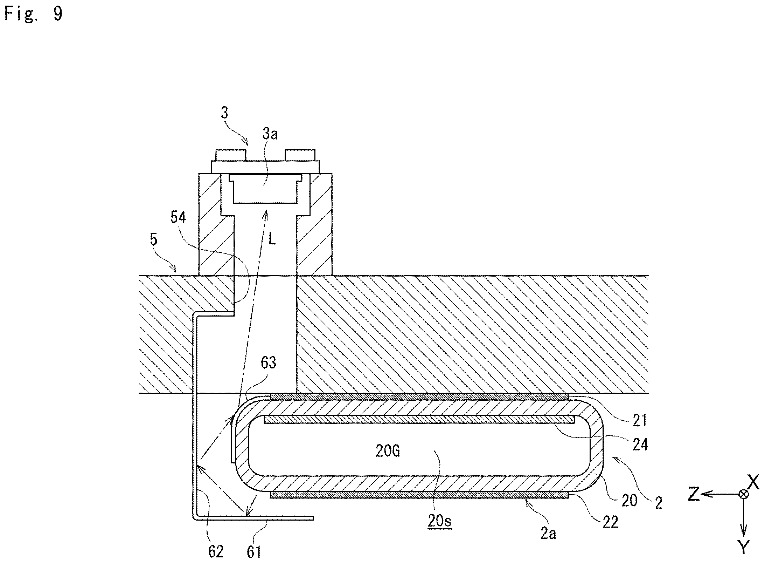

The structure adopted in each of the above embodiments can be adopted in any other embodiment. The specific configuration of each component is not limited only to that in the above-described embodiment, and various modifications are possible without departing from the spirit of the present invention.
(1) In the ultraviolet light emission device 1 according to the above embodiment, the excimer lamp 2 includes the light-emitting tube 20 having transparency to ultraviolet light, and the reflective film 24 formed on the inner wall surface of the light-emitting tube 20 and facing the light emission surface 2a across the tube axis 20c of the light-emitting tube 20. However, the ultraviolet light emission device 1 is not limited to this configuration. For example, the excimer lamp 2 may include a light-emitting tube 20 having transparency to ultraviolet light, and a reflecting mirror disposed along the outer wall surface of the light-emitting tube 20 and facing the light emission surface 2a across the tube axis 20c of the light-emitting tube 20.
(2) The ultraviolet light emission device 1 according to the above embodiment further includes the second reflection member 62 disposed around the excimer lamp 2 and located lateral to the light emission surface 2a, and ultraviolet light L reflected by the first reflection member 61 is reflected by the second reflection member 62 and enters the light intensity sensor 3. However, the ultraviolet light emission device 1 is not limited to this configuration. For example, the second reflection member 62 is not always necessary, and the ultraviolet light L reflected by the first reflection member 61 may directly enter the light intensity sensor 3 without providing the second reflection member 62. In this case, the light intensity sensor 3 may be disposed, for example, lateral to the excimer lamp 2 so that the ultraviolet light L reflected by the first reflection member 61 easily enters the light intensity sensor 3.
(3) The ultraviolet light emission device 1 according to the above embodiment further includes the third reflection member 63 disposed between the excimer lamp 2 and the light intensity sensor 3, and ultraviolet light L reflected by the second reflection member 62 is reflected by the third reflection member 63 and enters the light intensity sensor 3. However, the ultraviolet light emission device 1 is not limited to this configuration. For example, the third reflection member 63 is not always necessary, and the ultraviolet light L reflected by the second reflection member 62 may directly enter the light intensity sensor 3 without providing the third reflection member 63.
(4) In the ultraviolet light emission device 1 according to the above embodiment, the light intensity sensor 3 faces the first reflection member 61 across the excimer lamp 2. However, the ultraviolet light emission device 1 is not limited to this configuration. For example, the light intensity sensor 3 and the first reflection member 61 may be disposed around the excimer lamp 2 while being shifted by 90 degrees.
(5) In the ultraviolet light emission device 1 according to the above embodiment, the light emission surface 2a is the lower surface of the light-emitting tube 20, and the ultraviolet light emission device 1 includes a plurality of the excimer lamps 2 that is arranged in the lateral direction such that the tube axes 20c of the light-emitting tubes 20 are parallel to each other, and a plurality of the light intensity sensors 3 each of which is disposed above the corresponding one of the excimer lamps 2. However, the ultraviolet light emission device 1 is not limited to this configuration. The ultraviolet light emission device 1 may include only one set of the excimer lamp 2 and the light intensity sensor 3.
(6) In the ultraviolet light emission device 1 according to the above embodiment, the light-emitting tube 20 of the excimer lamp 2 is a double tube including the outer tube 20a and the inner tube 20b, but is not limited thereto. The light-emitting tube 20 of the excimer lamp 2 may be, for example, a flat tube as illustrated in Fig. 9. In Fig. 9, portions denoted by reference signs same as those in Figs. 1 to 8 represent components having structures or functions (effects) substantially similar to those described in the above embodiment, and the descriptions thereof will not be repeated. The light-emitting tube 20 has a substantially rectangular cross section. The excimer lamp 2 includes a pair of electrodes 21 and 22 facing each other and a reflective film 24. The electrodes 21 and 22 have a mesh pattern. In the embodiment illustrated in Fig. 9, the ultraviolet light L emitted from the light emission surface 2a is sequentially reflected by the first reflection member 61, the second reflection member 62, and the third reflection member 63, and enters the light intensity sensor 3.

### DESCRIPTION OF REFERENCE SIGNS

- 1: Ultraviolet light emission device
- 2: Excimer lamp
- 2a: Light emission surface
- 3: Light intensity sensor
- 3a: Light receiving unit
- 4: Conveyance mechanism
- 5: Cooling block
- 20: Light-emitting tube
- 20c: Tube axis
- 20s: Discharge space
- 21: First electrode
- 22: Second electrode
- 23: Lighting power supply
- 24: Reflective film
- 25: Control device
- 54: Through hole
- 61: First reflection member
- 62: Second reflection member
- 63: Third reflection member
- L: Ultraviolet light
- W: Object for irradiation

## Claims

1. An ultraviolet light emission device (1) comprising:
an excimer lamp (2) having an elongated shape and having a light emission surface (2a) that emits ultraviolet light (L) toward an object (W) for irradiation;
a light intensity sensor (3) that is disposed around the excimer lamp (2) and detects ultraviolet light (L); and
a first reflection member (61) disposed around the excimer lamp (2) and facing a part of the light emission surface (2a) in a longitudinal direction, wherein
ultraviolet light (L) emitted from the light emission surface (2a) is reflected by the first reflection member (61) and enters the light intensity sensor (3).

2. The ultraviolet light emission device (1) according to claim 1, wherein the excimer lamp (2) includes a light-emitting tube (20) having transparency to ultraviolet light (L), and a reflective film (24) formed on an inner wall surface of the light-emitting tube (20) and facing the light emission surface (2a) across a tube axis (20c) of the light-emitting tube (20).

3. The ultraviolet light emission device (1) according to claim 1 or 2, further comprising a second reflection member (62) disposed around the excimer lamp (2) and located lateral to the light emission surface (2a), wherein
ultraviolet light (L) reflected by the first reflection member (61) is reflected by the second reflection member (62) and enters the light intensity sensor (3).

4. The ultraviolet light emission device (1) according to claim 3, further comprising a third reflection member (63) disposed between the excimer lamp (2) and the light intensity sensor (3), wherein
ultraviolet light (L) reflected by the second reflection member (62) is reflected by the third reflection member (63) and enters the light intensity sensor (3).

5. The ultraviolet light emission device (1) according to claim 1 or 2, wherein the light intensity sensor (3) faces the first reflection member (61) across the excimer lamp (2).

6. The ultraviolet light emission device (1) according to claim 1 or 2, wherein a space between a periphery of the excimer lamp (2) and the light intensity sensor (3) has an inert gas atmosphere.

7. The ultraviolet light emission device (1) according to claim 3, wherein
the light emission surface (2a) is a lower surface of the light-emitting tube (20),
the ultraviolet light emission device (1) including a plurality of the excimer lamps (2) that is arranged in a lateral direction such that tube axes (20c) of the light-emitting tubes (20) are parallel to each other, and a plurality of the light intensity sensors (3) each of which is disposed above the corresponding one of the excimer lamps (2).

8. The ultraviolet light emission device (1) according to claim 7, wherein the second reflection member (62) is disposed between the excimer lamps (2) adjacent to each other.
